# EUROPEAN PATENT SPECIFICATION

(11) **EP 3 679 973 B1**
(45) Date of publication and mention of the grant of the patent: **22.06.2022**
(21) Application number: 17924029.6
(22) Date of filing: 06.09.2017
(51) Int. Cl.: A61M 16/00, A61M 16/04, A61B 1/24, A61B 16/00, A61C 17/10

(54) **ORAL NOZZLE FOR ENDOSCOPY PROCEDURES**
MUNDDÜSE FÜR ENDOSKOPIEVERFAHREN
EMBOUT BUCCAL POUR PROCÉDÉS ENDOSCOPIQUES

(43) Date of publication of application: 15.07.2020
(73) Proprietor: Muñiz Herrera, Cristián Clemente, Santiago (CL)
(72) Inventor: Muñiz Herrera, Cristián Clemente, Santiago (CL)
(74) Representative: Garcia González, Sergio
(86) International application number: PCT/CL2017/050050
(87) International publication number: WO 2019/046980

(56) References cited:
- WO-A1-2005/016142
- WO-A1-2011/103543
- WO-A1-2014/121199
- WO-A2-2007/061609
- JP-A- 2010 240 191
- KR-Y1- 200 460 724
- US-A1- 2007 006 878
- US-A1- 2010 317 987
- US-A1- 2014 209 095

## Description

### FIELD OF THE INVENTION

The present invention belongs to the medical device industry, particularly to the field of disposable oral nozzles used in the procedures performed endoscopically, where a camera or similar device is introduced orally to either the airway or the patient's digestive tract, such as Upper Digestive Endoscopy, Endosonography, Fibrobronchoscopy, Trans Esophageal Echocardiography, among others.

### BACKGROUND OF THE INVENTION

In endoscopic procedures such as those mentioned above, the use of generally disposable plastic nozzles that are inserted into the patient's mouth both vigil and sedated or anesthetized, in order to keep the oral cavity open and allow the endoscope to pass through it into the oropharynx and esophagus or larynx and trachea is recurrent, and then into the anatomical cavities to be examined, preventing the patient from involuntarily biting the endoscope by blocking it, damaging it and hindering the medical procedure.

Generally, the nozzles for endoscopic procedures consist of a solid body of generally oval section whose surface allows contact with the denture or gums of the patient in an atraumatic way and on whose anterior face a surface perpendicular to said contact surface is formed, which rests against the patient's lips to prevent the nozzle from being swallowed during the medical procedure. Usually, the nozzle has hooking means at its lateral ends that allow connection with an elastic band that is positioned around the patient's neck, in order to prevent the nozzle from detaching or being extruded or involuntarily displaced from its position inside the mouth by the sedated or anesthetized patient.

An example of this type of nozzles is that disclosed by document US 5174284, which describes an annular shaped plastic nozzle whose back face arises an arched upward projection that improves the access area of the endoscope, maintaining the mouth, teeth and the jaws substantially immobilized.

A drawback of the aforementioned device has to do with the fact that they do not allow the administration of oxygen to the patient during the endoscopic procedure, which is known to be necessary to avoid hypoxia, that is, the undesirable drop in the level of blood oxygen saturation produced by hypoventilation during sedation and the physical presence of the endoscope in the esophagus or the fibrobronchoscope in the trachea. In order to avoid the above, it has been proposed to combine this type of nozzles with the use of nasal cannulas, with masks adapted or even perforated by hand to allow the passage of the endoscope to and through the oral cavity. However, these solutions are usually inefficient in the first place because they are uncomfortable for the endoscopist, involving the use of many devices in a small space that limit the movements required for the medical procedure to be performed and secondly because they involve a high cost in medical devices which end up damaging and making their function more inefficient, and wasting them on purposes for which they are not conceived, as happens for example in the case of oxygen masks or nasal cannulas, the latter also having the disadvantage that at being very flexible they bend and detach from their position in the patient's nose. In turn, facial masks (Venturi or Campbell type), when cut and losing their original architecture, also move more easily from their correct position, causing damage to the patient such as, for example, corneal injuries by rubbing the eyes on the sedated or anesthetized patient.

In order to address these drawbacks, document US 5273032 proposes an oral apparatus of the type in question with conduit means for directing at least one gas stream into the hole of the apparatus and at least another gas stream to the air breathed through of the patient's nose. To achieve this, the nozzle comprises on its front face a distribution conduit that is divided into two open vertical branches to direct the gas towards the patient's nostrils and two horizontal branches that extend in the direction of the oral cavity through the body of the nozzle, the distribution conduit being configured to connect with a conventional plastic tube for oxygen supply. Other similar devices are disclosed in documents US 5513634 and US 9186473 which allow the administration of oxygen by means of nasal conduits of the nozzle or by the anchoring of standard nasal cannulas to the body of the nozzle.

A drawback of the devices described above has to do mainly with the disadvantages involved in the supply of oxygen to the patient exclusively by the nasal route in procedures that involve sedation, since the flow should be restricted to 3-4 liters/minute, since above that, it becomes unpleasant for the patient and the turbulent and dry flow that impacts the nasal mucosa makes it impossible for it to be used clinically. On the other hand, in the vast majority of patients in states of impaired consciousness such as sedation or anesthesia, dysfunction or fall of the palate veil occurs, which causes patients during endoscopic procedures to ventilate exclusively through the mouth, leaving the nasal cavity and naso-pharynx, clogged and not participating in ventilation.

To deal with these drawbacks, some nozzles have been proposed in the prior art that allow the patient to be supplied with oxygen through a conduit exclusively for the oral route, such as those described in documents WO 2005016142 , WO2014121199 and US 2015265792.

In particular, document WO 2005016142 proposes a nozzle with a removable gas distributor comprising at least one nasal outlet orifice and an oral outlet orifice that is connected with an elongated opening located in the upper inner part of the nozzle to direct the gas to the patient's mouth.

For its part, document US 2015265792 discloses an oral nozzle for endoscopic procedures consisting of a body with an arched flange on its upper face including a first tubular element that crosses it for insertion of the endoscope into the patient's mouth and a second tubular element that defines a second channel inclined with respect to the flange and which serves to maintain an airway and ventilate the patient through the mouth by means of the connection with a breathing apparatus.

Although the devices proposed by the two aforementioned references allow to provide a flow of air directly into the patient's mouth, this is provided only through a small hole that provides a high resistance to flow, which disadvantageously generates that the fluid is supplied in the form of a jet, causing discomfort to the patient in the area of the mouth where said jet impacts.

Another drawback with these devices is that when the flow of oxygen is delivered through a single duct, the risks of clogging it by secretions are very high, especially considering that the patient in a state of sedation or anesthesia could involuntarily cover said duct with the tongue and therefore block the oxygen supply.

It is also noted that the aforementioned devices are of a complex construction and often require special connections with the fluid supply devices and not those traditionally used in an operating room. In some cases they even have moving and removable parts that could be lost and that hinder the cleaning operation when they are not disposable, not to mention the high costs involved in its construction.

It is therefore the objective of the present invention to overcome the inconveniences observed in the prior art by providing an oral nozzle for endoscopic procedures that, together with allowing the operation of the endoscope, it allows to deliver a gas flow directly and exclusively into the patient's mouth with no limit of liters per minute more than what the used oxygen source can deliver through a wide and low resistance outlet, preventing said flow from affecting the patient and/or being obstructed.

It is another objective of the present invention to provide an oral nozzle for endoscopic procedures that is manufactured in one piece, without moving parts and that supports standard connections with external sources, providing a simple, inexpensive and intuitive construction and design in its use for the operator.

### DESCRIPTION OF THE INVENTION

The nozzle proposed herein preferably consists of a one-piece body formed of plastic material, which comprises a central tubular body whose interior forms a passage that crosses the nozzle throughout its entire length. Preferably the central body has an oval shape, so that when inserted horizontally in the patient's mouth, its surface allows the support of the teeth or gums in the toothless patients, on it.

In turn, at the ends of the central body, flanges are configured that limit the movement of the nozzle into the patient's mouth. Thus, the central flange has an oval shape with a height significantly greater than the central body, which prevents the nozzle from being swallowed by the patient as it acts as a barrier that rests against the patient's lips. Similarly, the lower posterior flange allows the nozzle to enter the patient's mouth but prevents it from moving too far forward and out of the mouth, when bumping with the teeth or gums when they are in contact with the surface of the central body.

From one of the lateral ends of the nozzle, a cylindrical shaped flow intake duct emerges, which can be connected to any source of oxygen supply or other gas or gas mixture, by means of a silicone hose, thanks to its diameter of standard dimensions for connection with said type of hoses. Advantageously, the location of the conduit at the side of the central body avoids elements in the anterior area of the nozzle that could interfere with the endoscope or hinder its operation by the endoscopist.

The flow intake duct is connected to a flow distributor channel consisting of a chamber located inside the central body and concentric to it, so that the chamber opening is distributed over the front face of the nozzle and allows the administration within the patient's mouth of a constant, homogeneous and concentrically distributed flow to the nozzle, avoiding a focused admission and therefore, the risks that it can be covered or to cause discomfort to the patient.

According to an alternative embodiment of the invention, the nozzle comprises a cylindrical shaped flow extraction conduit connected to the central body, to allow capnography measurement (exhaled CO₂) when the nozzle is in the patient's mouth during the endoscopy procedure. For this, the flow extraction duct is located in a position closer to the rear end of the nozzle and is connected to a conductive passage disposed inside the central body and in communication with a flow extraction opening in the rear face of the nozzle. In this way, part of the flow exhaled through the patient's mouth enters through the flow extraction opening and travels through the conductive passage to exit through the extraction conduit.

Preferably, the extraction conduit has a diameter suitable for standard Lüer connections and is located parallel to the flow intake conduit to avoid disturbing the operation of the endoscope.

According to alternative embodiments of the invention, the nozzle comprises at its lateral ends suitable coupling means to allow connection with an elastic band that is positioned around the neck of the patient.

From the described configuration, it is possible to obtain a disposable plastic nozzle that does not comprise moving parts, providing a flow of air supplied with high volumes per minute, the configuration of which is simple and can be manufactured at a very low cost.

These and other embodiments can be seen in greater detail from the accompanying Figures and described below.

### DESCRIPTION OF THE FIGURES

- Figure 1 illustrates a top perspective view of the nozzle of the present invention.
- Figure 2 illustrates a bottom perspective view of the nozzle of the present invention.
- Figure 3 illustrates a sectional view of the nozzle of the present invention.
- Figure 4 illustrates a profile view of the nozzle according to a second embodiment.
- Figure 5 illustrates a bottom view of the nozzle according to a second embodiment.

### DETAILED DESCRIPTION OF THE INVENTION

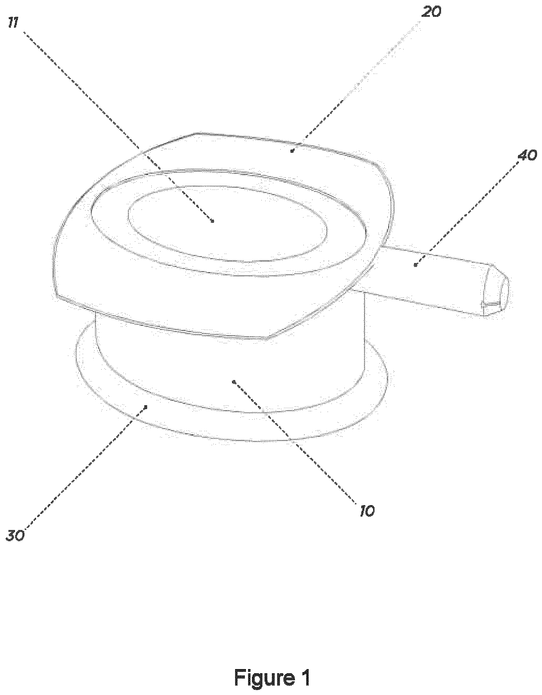

As seen in Figure 1, the nozzle of the present invention is configured from a central tubular body 10 within which a passage 11 is formed, crossing the nozzle in its entire length. According to the illustrated embodiment, the central body 10 has an oval shape.

At the front end of the central body 10, an anterior oval shaped flange 20 with straight ends and softened edges is configured, located in the opposite direction to the central body 10, said front flange being preferably curved towards the rear face of the nozzle. On the other hand, at the rear end of the central body 10 a rear flange 30 of a width substantially smaller than the anterior flange and of a preferably constant diameter is configured.

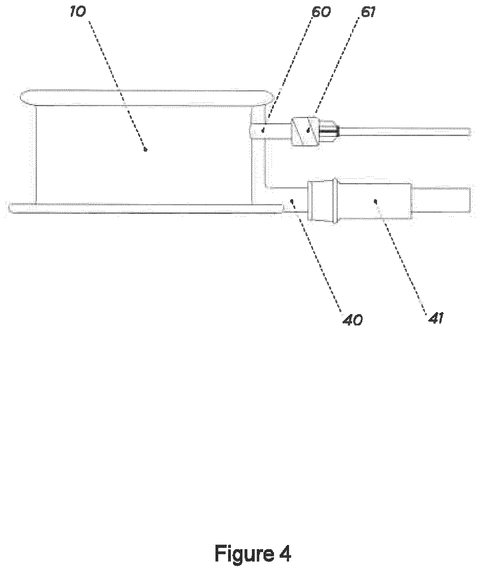

From one of the lateral ends of the nozzle, emerges a cylindrical flow inlet conduit 40, whose outer diameter is preferably 7 mm, configured for the connection of a silicone hose 41 (see Figure 4).

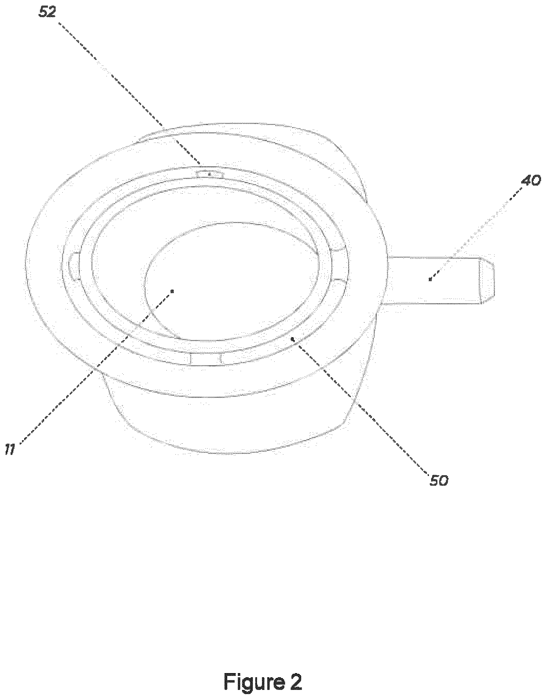

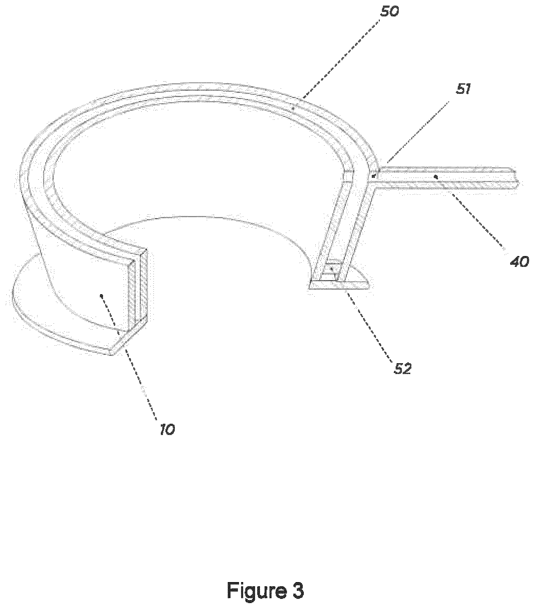

As seen in Figures 2 and 3, the flow intake conduit 40 is communicated with a flow distributor channel 50 consisting of a chamber located inside the central body 10 and concentric thereto, so that the opening of the chamber is distributed on the front face of the nozzle, around the passage 11. The communication between the flow distributor channel 50 and the flow intake conduit 40, as seen in Figure 3, occurs through a circular perforation 51 located inside the nozzle, particularly on the outer face of the flow distributor channel 50. Also, according to the illustrated embodiment, preferably between the inner and outer wall of the flow distributor channel 50 there is at least one reinforcing element 52 for stiffening the structure of said channel.

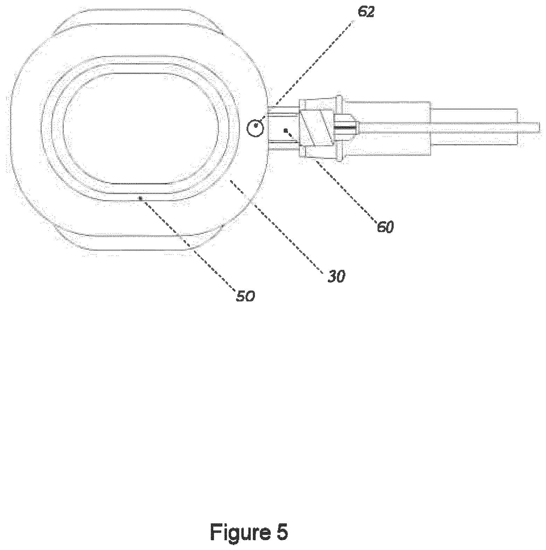

According to an alternative embodiment of the invention illustrated in Figures 4 and 5, the nozzle comprises a cylindrical shaped flow extraction conduit 60 connected to the central body 10, preferably smaller in diameter than the flow intake conduit 40 and located in a position closer to the rear end of the nozzle. According to the illustrated embodiment, the flow extraction conduit 60 has an outside diameter configured for a Lüer connection 61.

As seen in Figure 5, the flow extraction conduit 60 is connected to a conductive passage disposed inside the central body, preferably between the outer face of the flow distributor channel 50 and the outer face of the central body 10, forming a flow extraction opening 62 on the rear face of the nozzle, preferably on the rear flange 30.

## Claims

1. Oral nozzle for endoscopic procedures to deliver a high gas flow into the patient's mouth through a wide and low resistance outlet, preventing said flow from affecting the patient and/or being blocked, the nozzle comprising:
a tubular central body (10) with a passage (11);
an anterior flange (20) and a posterior flange (30);
a flow intake duct (40);
wherein said flow intake duct (40) is communicated by means of a perforation (51) located inside the nozzle with a flow distributor channel (50), said flow intake duct (40) being perpendicular to the flow distributor channel (50),
wherein the flow distributor channel consists of a chamber located inside the central body (10) and concentric to this, wherein the opening of said chamber is distributed over the front face of the nozzle and allows the administration within the patient's mouth of a flow, said opening being located completely around the passage (11).

2. The nozzle according to claim 1, wherein the perforation (51) is located on the outer face of the flow distributor channel (50).

3. The nozzle according to any of the preceding claims, wherein the flow intake passage (40) extends from one of the lateral ends of the nozzle.

4. The nozzle according to any of the preceding claims, wherein the flow intake passage (40) has a cylindrical shape with an outer diameter of 7 mm.

5. The nozzle according to any of the preceding claims, wherein it comprises a flow extraction duct (60) connected to the central body (10).

6. The nozzle according to claim 5, wherein the flow extraction duct (60) is connected to a conductive passage disposed inside the central body (10), comprising a flow extraction opening (62) on the rear face of the nozzle.

7. The nozzle according to claim 6, wherein the flow extraction opening (62) is located on the rear flange (30).

8. The nozzle according to any of claims 5 to 7 wherein the flow extraction duct (60) is cylindrical in shape and is configured for a Lüer connection (61).

9. The nozzle according to any of the preceding claims, wherein at least one reinforcing element (52) is arranged between the inner and outer wall of the flow distributor channel (50).

10. The nozzle according to any of the preceding claims, wherein the anterior flange (20) is oval and curved towards the rear face of the nozzle.

11. The nozzle according to any of the preceding claims, wherein the rear flange (30) has a width smaller than the anterior flange (20), with a constant diameter.

12. The nozzle according to any of the preceding claims, wherein the central body (10) has an oval shape

13. The nozzle according to any of the preceding claims, wherein it comprises coupling means for connection with an elastic band at its lateral ends.

## Patentansprüche

1. Oraldüse für endoskopische Verfahren zur Abgabe eines hohen Gasflusses in den Mund des Patienten durch einen breiten und widerstandsarmen Auslass, wobei verhindert wird, dass der Durchfluss den Patienten beeinträchtigt und/oder blockiert wird, wobei die Düse umfasst:
einen rohrförmigen zentralen Körper (10) mit einem Durchgang (11);
einen vorderen Flansch (20) und einen hinteren Flansch (30);
eine Durchflusseinlassleitung (40);
wobei die Durchflusseinlassleitung (40) mittels einer Perforation (51), die sich im Inneren der Düse befindet, mit einem Durchflussverteilerkanal (50) in Verbindung steht, wobei die Durchflusseinlassleitung (40) senkrecht zum Durchflussverteilerkanal (50) ist,
wobei der Durchflussverteilerkanal aus einer Kammer besteht, die sich im Inneren des zentralen Körpers (10) befindet und konzentrisch zu diesem ist, wobei die Öffnung der Kammer über die Vorderseite der Düse verteilt ist und die Verabreichung eines Durchflusses in den Mund des Patienten ermöglicht, wobei die Öffnung vollständig um den Durchgang (11) herum angeordnet ist.

2. Düse nach Anspruch 1, wobei sich die Perforation (51) an der Außenseite des Durchflussverteilerkanals (50) befindet.

3. Düse nach einem der vorhergehenden Ansprüche, wobei sich die Durchflusseinlassleitung (40) von einem der seitlichen Enden der Düse aus erstreckt.

4. Düse nach einem der vorhergehenden Ansprüche, wobei die Durchflusseinlassleitung (40) eine zylindrische Form mit einem Außendurchmesser von 7 mm aufweist.

5. Düse nach einem der vorhergehenden Ansprüche, wobei sie eine Durchflussauslassleitung (60) umfasst, die mit dem zentralen Körper (10) verbunden ist.

6. Düse nach Anspruch 5, wobei die Durchflussauslassleitung (60) mit einem im Inneren des zentralen Körpers (10) angeordneten Leitungsdurchgang verbunden ist, der an der Rückseite der Düse eine Durchflussauslassöffnung (62) umfasst.

7. Düse nach Anspruch 6, wobei sich die Durchflussauslassöffnung (62) am hinteren Flansch (30) befindet.

8. Düse nach einem der Ansprüche 5 bis 7, wobei die Durchflussauslassleitung (60) zylindrisch ist und für einen Lüer-Anschluss (61) ausgebildet ist.

9. Düse nach einem der vorhergehenden Ansprüche, wobei zwischen der Innen- und Außenwand des Durchflussverteilerkanals (50) mindestens ein Verstärkungselement (52) angeordnet ist.

10. Düse nach einem der vorhergehenden Ansprüche, wobei der vordere Flansch (20) oval und zur Rückseite der Düse hin gebogen ist.

11. Düse nach einem der vorhergehenden Ansprüche, wobei der hintere Flansch (30) eine geringere Breite als der vordere Flansch (20) und einen konstanten Durchmesser aufweist.

12. Düse nach einem der vorhergehenden Ansprüche, wobei der zentrale Körper (10) eine ovale Form aufweist.

13. Düse nach einem der vorhergehenden Ansprüche, **dadurch gekennzeichnet, dass** sie an ihren seitlichen Enden Verbindungsmittel zur Verbindung mit einem elastischen Band aufweist.

## Revendications

1. Buse orale pour des procédures endoscopiques destinée à délivrer un écoulement de gaz élevé dans la bouche du patient à travers une sortie large et à faible résistance, empêchant ledit écoulement d'affecter le patient et/ou d'être bloqué, la buse comprenant :
un corps central tubulaire (10) avec un passage (11) ;
une bride antérieure (20) et une bride postérieure (30) ;
un conduit d'entrée d'écoulement (40) ;
ledit conduit d'entrée d'écoulement (40) étant mis en communication au moyen d'une perforation (51) située à l'intérieur de la buse avec un canal de distribution d'écoulement (50), ledit conduit d'entrée d'écoulement (40) étant perpendiculaire au canal de distribution d'écoulement (50),
le canal de distribution d'écoulement étant constitué d'une chambre située à l'intérieur du corps central (10) et concentrique à celui-ci, l'ouverture de ladite chambre étant répartie sur la face avant de la buse et permettant l'administration à l'intérieur de la bouche du patient d'un écoulement, ladite ouverture étant située entièrement autour du passage (11).

2. Buse selon la revendication 1, dans laquelle la perforation (51) est située sur la face externe du canal de distribution d'écoulement (50).

3. Buse selon l'une quelconque des revendications précédentes, dans laquelle le passage d'entrée d'écoulement (40) s'étend depuis l'une des extrémités latérales de la buse.

4. Buse selon l'une quelconque des revendications précédentes, dans laquelle le passage d'entrée d'écoulement (40) a une forme cylindrique avec un diamètre externe de 7 mm.

5. Buse selon l'une quelconque des revendications précédentes, dans laquelle elle comprend un conduit d'extraction d'écoulement (60) relié au corps central (10).

6. Buse selon la revendication 5, dans laquelle le conduit d'extraction d'écoulement (60) est relié à un passage conducteur disposé à l'intérieur du corps central (10), comprenant une ouverture d'extraction d'écoulement (62) sur la face arrière de la buse.

7. Buse selon la revendication 6, dans laquelle l'ouverture d'extraction d'écoulement (62) est située sur la bride arrière (30).

8. Buse selon l'une quelconque des revendications 5 à 7, dans laquelle le conduit d'extraction d'écoulement (60) est de forme cylindrique et est configuré pour un raccord Lüer (61).

9. Buse selon l'une quelconque des revendications précédentes, dans laquelle au moins un élément de renfort (52) est agencé entre les parois interne et externe du canal de distribution d'écoulement (50).

10. Buse selon l'une quelconque des revendications précédentes, dans laquelle la bride antérieure (20) est ovale et incurvée vers la face arrière de la buse.

11. Buse selon l'une quelconque des revendications précédentes, dans laquelle la bride arrière (30) a une largeur plus petite que la bride antérieure (20), avec un diamètre constant.

12. Buse selon l'une quelconque des revendications précédentes, dans laquelle le corps central (10) a une forme ovale.

13. Buse selon l'une quelconque des revendications précédentes, dans laquelle elle comprend un moyen d'accouplement pour un raccord avec une bande élastique à ses extrémités latérales.
